# EUROPÄISCHE PATENTSCHRIFT

(11) **EP 2 298 244 B1**
(45) Veröffentlichungstag und Bekanntmachung des Hinweises auf die Patenterteilung: **31.05.2017**
(21) Anmeldenummer: 09012035.3
(22) Anmeldetag: 22.09.2009
(51) Int. Cl.: A61F 2/36, A61F 2/30, A61F 2/32, A61F 2/34

(54) **Gelenkprothese mit Gelenkkapsel**
Joint prosthesis with joint capsule
Prothèse d'articulation avec capsule articulaire

(43) Veröffentlichungstag der Anmeldung: 23.03.2011
(73) Patentinhaber: Wolter, Dietmar, 22955 Hoisdorf (DE)
(72) Erfinder: Wolter, Dietmar, 22955 Hoisdorf (DE)
(74) Vertreter: Glawe, Delfs, Moll

(56) Entgegenhaltungen:
- EP-A- 1 057 461
- WO-A-2007/125060
- DE-A- 2 501 080
- DE-A- 10 328 949
- DE-A- 10 339 605
- US-A- 3 683 421
- US-A- 4 467 479
- US-A- 5 514 182
- US-A1- 2003 229 398
- US-A1- 2009 036 995
- US-B1- 7 476 250

## Beschreibung

Die Erfindung betrifft eine Gelenkkapselprothese, die eine erste Prothesenkomponente und eine zweite Prothesenkomponente umfasst. Die Prothesenkomponenten wirken über Gelenkflächen zusammen und bilden ein Gleitgelenk. Die Gelenkprothese umfasst eine Gelenkkapsel, die sich von der ersten Prothesenkomponente zu der zweiten Prothesenkomponente erstreckt und die das Gleitgelenk umschließt. Gelenkprothesen werden beim Menschen oder auch bei Tieren eingesetzt, wenn das natürliche Gelenk nicht mehr funktionsfähig ist. Die beiden Prothesenkomponenten werden mit den an das Gelenk angrenzenden Knochen verbunden. Das Gleitgelenk bildet die Funktion des natürlichen Gelenks nach. Bei jeder Bewegung der Gelenkprothese wird durch Reibung Material in Form von feinen Partikeln von den Gelenkflächen abgetragen. Das abgetragene Material verteilt sich in dem umliegenden Körpergewebe und hat dort unerwünschte Wirkungen. Die Partikel werden als wesentlicher Grund dafür angesehen, dass sich die Verbindung zwischen den Prothesenkomponenten und dem Knochen im Laufe der Zeit lockert. Bekannt ist es, das Gleitgelenk mit einer Gelenkkapsel zu umgeben, so dass die feinen Partikel daran gehindert werden, in das umliegende Körpergewebe einzudringen, EP 1 057 461. Die US 2003/0229398 (Basis für den Oberbegriff des Anspruchs 1) offenbart eine mehrschichtige künstliche Gelenkkapsel. Bei den bislang bekannten Gelenkprothesen reibt die Gelenkkapsel am umliegenden Körpergewebe, wenn das Gelenk bewegt wird. Die Folge können Reizungen des Gewebes und Schmerzen für den Patienten sein.

Der Erfindung liegt die Aufgabe zu Grunde, eine Gelenkprothese vorzustellen, die für den Patienten besser verträglich ist. Ausgehend vom eingangs genannten Stand der Technik wird die Aufgabe gelöst durch die Merkmale des Anspruchs 1. Erfindungsgemäß ist in einem Flächenbereich der Gelenkkapsel eine doppelte Wand vorgesehen, in der eine Innenschicht und eine Außenschicht parallel zueinander verschiebbar sind. Als parallel wird eine Verschiebung zweier Schichten zueinander bezeichnet, wenn der Abstand der beiden Schichten zueinander während der Verschiebung im Wesentlichen konstant bleibt. Vorteilhafte Ausführungsformen finden sich in den Unteransprüchen.

Zunächst werden einige Begriffe erläutert. Die Gelenkprothese kann zum Ersatz eines beliebigen Gelenks des menschlichen oder tierischen Skeletts bestimmt sein. In einem häufigen Anwendungsfall ist die Gelenkprothese eine Hüftprothese. Die Prothesenkomponenten werden mit an das zu ersetzende Gelenk angrenzenden Knochen verbunden und bilden über das Gleitgelenk die Funktion des natürlichen Gelenks nach. Die Prothesenkomponenten entsprechen in ihrer Form und Funktion den üblichen und bekannten Gelenkprothesen. Die Prothesenkomponenten können beispielsweise aus Metallen, wie geschmiedeten Stählen oder Titanlegierungen, aus Kunststoff oder Keramik bestehen. In dem Gleitgelenk können gleiche oder unterschiedliche dieser Materialien zusammenwirken.

Die Gelenkkapsel umschließt das Gleitgelenk, so dass Partikel, die von den Gleitflächen abgetragen werden, von dem umliegenden Körpergewebe fern gehalten werden. Das eine Ende der Gelenkkapsel ist mit der ersten Prothesenkomponente verbunden, das andere Ende der Gelenkkapsel ist mit der zweiten Prothesenkomponente verbunden. Die Verbindung ist jeweils dicht, so dass die Partikel in dem von der Gelenkkapsel umschlossenen Raum gehalten werden und nicht austreten können. Zwischen den beiden Prothesenkomponenten bildet die Gelenkkapsel eine geschlossene Hülle um das Gleitgelenk herum.

Bei der erfindungsgemäßen Gelenkkapsel kann die Außenschicht der doppelten Wand eine Verbindung mit dem umliegenden Körpergewebe eingehen, so dass die Außenschicht sich bei einer Bewegung des Gelenks nicht relativ zum Körpergewebe bewegt. Die Innenschicht kann einer Bewegung des Gelenks folgen und sich dabei parallel zur Außenschicht verschieben. Die Bewegung der Innenschicht führt also nicht zu Reibung zwischen der Innenschicht und dem Körpergewebe. Das Risiko, dass das Körpergewebe gereizt wird, ist geringer.

Erfindungsgemäß ist die Doppelwandigkeit auf einem Flächenbereich und damit mindestens auf einem Teil der Oberfläche der Gelenkkapsel vorgesehen. Vorzugsweise erstreckt sich die Doppelwandigkeit über mindestens 50%, weiter vorzugsweise mindestens 70% der Wand der Gelenkkapsel. Die Doppelwandigkeit ist vorzugsweise in dem Bereich vorgesehen, in dem die Gelenkkapsel den Zwischenraum zwischen den beiden Prothesenkomponenten überbrückt, also in dem die Gelenkkapsel nicht fest mit einer der Prothesenkomponenten verbunden ist. Möglich ist es auch, dass die Gelenkkapsel insgesamt doppelwandig ist.

Künstliche Gelenkkapseln sollen allgemein das Gleitgelenk möglichst eng umgeben, damit die Gelenkkapsel nur wenig Platz im Körper einnimmt. Allerdings besteht bei einer engen Ausführung die Gefahr, dass die Gelenkkapsel zwischen den beiden Gelenkflächen des Gleitgelenks eingeklemmt wird und dadurch Schaden nimmt. Im Rahmen der Erfindung soll dies durch eine Gestaltung vermieden werden, bei der die Wand der Gelenkkapsel einer Bewegung des Gelenks immer nach außen ausweicht. Erreicht werden kann dies dadurch, dass die Außenschicht sich aufgrund ihrer Form nur nach außen wölben kann und dass die Außenschicht die Innenschicht mit nach außen zieht.

Um sicherzustellen, dass die Außenschicht sich bei einer Bewegung des Gleitgelenks nach außen wölbt, kann die Außenschicht mit Verstärkungsstreifen versehen sein. Als Verstärkungsstreifen wird ein Bereich der Außenschicht bezeichnet, in dem die Materialstärke größer ist und der damit steifer ist als der Rest der Außenschicht. Im Bereich der Verstärkungsstreifen hat die Außenschicht vorzugsweise eine konvexe Form. Beispielsweise kann der Verstärkungsstreifen sich in Umfangsrichtung als Ring um das Gleitgelenk herum erstrecken. Alternativ oder zusätzlich dazu können ein oder mehrere Verstärkungsstreifen vorgesehen sein, die sich von einem Ende der Gelenkkapsel zum anderen Ende der Gelenkkapsel erstrecken und damit unter einem Winkel oder rechtwinklig zur Umfangsrichtung ausgerichtet sind. Sind die Verstärkungsstreifen unter einem von 90° verschiedenen Winkel zur Umfangsrichtung ausgerichtet, sind sie besonders geeignet, um auch Twistbewegungen des Gelenks aufzunehmen. Die Fähigkeit zur Aufnahme von Twistbewegungen kann außerdem dadurch verbessert werden, dass die Verstärkungsstreifen nicht geradlinig sind, sondern eine gebogene Form, wie beispielsweise eine S-Form haben. Die Wirksamkeit der Verstärkungsstreifen kann erhöht werden, wenn die Dicke der Verstärkungsstreifen im Zentrum größer ist als nahe den Enden der Gelenkkapsel. Im Querschnitt kann der Verstärkungsstreifen sich von der Basis zum freien Ende hin verjüngen. Beispielsweise kann der Verstärkungsstreifen im Querschnitt dreieckig sein, wobei die Ecken des Dreiecks abgerundet sein können.

Wenn die Außenschicht sich nach außen wölbt, soll die Innenschicht dieser Bewegung folgen. Vorzugsweise wird dies dadurch erreicht, dass die Außenschicht eine Zugkraft auf die Innenschicht überträgt. In einer vorteilhaften Ausführungsform haften die Innenschicht und die Außenschicht dadurch ausreichend aneinander, dass sie zumindest teilweise flächig aufeinander liegen. Die Adhäsionskräfte zwischen den Schichten sind dann groß genug, dass die Innenschicht einer nach außen gerichteten Bewegung der Außenschicht folgt. Um sicherzustellen, dass die Schichten sich in Parallelrichtung trotzdem leicht relativ zueinander verschieben lassen, kann die Außenschicht und/oder die Innenschicht auf der entsprechenden Seite mit einer reibungsmindernden Beschichtung versehen sein.

Die Reibung zwischen der Innenschicht und der Außenschicht kann auch dadurch klein gehalten werden, dass eine Flüssigkeit oder ein Gel in den Zwischenraum eingebracht wird. Um trotzdem eine hinreichende Haftung zwischen der Innenschicht und der Außenschicht zu haben, ist die von der Flüssigkeit oder dem Gel gebildete Schicht vorzugsweise so dünn, dass die Innenschicht und die Außenschicht kapillar aneinander haften. Wölbt sich die Außenschicht bei einer Bewegung der Gelenkprothese nach außen, so wird durch die kapillare Haftung auch die Innenschicht nach außen gezogen.

Bei einem größeren Abstand, bei dem die Schichten nicht mehr unmittelbar aneinander haften, können ein oder mehrere Zugelemente zwischen der Innenschicht und der Außenschicht vorgesehen sein. Durch die Zugelemente wird die Innenschicht nach außen gezogen, wenn die Außenschicht sich nach außen wölbt. Gegebenenfalls können die Innenschicht und die Außenschicht punktuell miteinander verklebt sein, um Zugkräfte zu übertragen.

Für die Verbindung zum Prothesenelement kann an einem Ende der Gelenkkapsel ein Wulst ausgebildet sein, der in eine Vertiefung des Prothesenelements eingreift. Der Wulst ist vorzugsweise so bemessen, dass er unter Spannung in der Vertiefung sitzt. Gegenüber den in erster Linie auftretenden Zugkräften ist die Gelenkkapsel dann sicher an dem Prothesenelement fixiert. Vorzugsweise ist an beiden Enden der Gelenkkapsel ein Wulst ausgebildet, so dass ein Wulst mit dem ersten Prothesenelement und der andere Wulst mit dem zweiten Prothesenelement verbunden ist.

Der Wulst kann eine Verdickung in dem Material der Gelenkkapsel sein. Möglich ist es auch, den Wulst dadurch zu bilden, dass das Ende der Gelenkkapsel aufgerollt wird. Ausreichen kann es, wenn entweder die Innenschicht oder die Außenschicht aufgerollt wird. In einer vorteilhaften Ausführungsform wird die Innenschicht zusammen mit der Außenschicht aufgerollt, so dass zugleich der Raum zwischen den Schichten zu allen Seiten hin dicht verschlossen ist. Eine Flüssigkeit oder ein Gel kann auf diese weise sicher im Zwischenraum gehalten werden. Alternativ oder zusätzlich können die Innenschicht und die Außenschicht an den Enden miteinander verklebt sein.

Die Gelenkkapsel ist vorzugsweise derart gestaltet, dass ihre Wand unter einer leichten Spannung steht, wenn die beiden Prothesenkomponenten eine Mittelposition zueinander einnehmen. Wird das Gelenk bewegt, wird die Spannung in der Wand der Gelenkkapsel auf der einen Seite erhöht und auf der anderen Seite vermindert. Vorzugsweise ist das Material der Gelenkkapsel derart elastisch dehnbar, dass es nach einer solchen Bewegung wieder in seinen Ausgangszustand zurückkehrt. In Betracht als Material für die Gelenkkapsel kommen beispielsweise Silikonverbindungen, wie sie seit den 1960er Jahren für Brustimplantate verwendet werden.

Erfindungsgemäß soll die Außenschicht der Gelenkkapsel eine Verbindung mit dem umliegenden Körpergewebe eingehen, während die Innenschicht sich relativ zur Außenschicht bewegen soll, ohne das umliegende Körpergewebe zu reizen. Um das Anwachsen des Körpergewebes zu erleichtern, kann die Außenschicht auf ihrer Außenseite eine strukturierte Oberfläche aufweisen. Die Struktur kann in Form einer Beschichtung auf die Außenschicht aufgebracht sein. Die Flexibilität der Außenschicht soll durch die Beschichtung möglichst nicht beeinträchtigt werden. Erreicht werden kann dies, indem die Beschichtung in Form eines Pulvers oder in Form von Mikropartikeln aufgebracht wird. Beispielsweise können die Partikel Polylactide oder Polyglykolide sein. Auch ein inerter Stoff wie beispielsweise Keramikstaub kommt für die Beschichtung in Betracht. Die Beschichtung kann eine Nanobeschichtung sein.

Bei einigen Gelenkprothesen besteht eine der Gelenkflächen aus einem Keramikmaterial. Keramikmaterial ist spröde und kann in viele kleine Bruchstücke zersplittern. Wenn die Gelenkfläche einer Prothese zersplittert, verteilen sich die Bruchstücke im umliegenden Körpergewebe und müssen aufwändig einzeln herausoperiert werden. Die erfindungsgemäße Gelenkprothese ist vorzugsweise so gestaltet, dass die künstliche Gelenkkapsel den Austritt solcher Bruchstücke verhindert. Dazu kann eine der Schichten verstärkt sein, so dass sie von den Bruchstücken nicht durchtrennt werden kann. Häufig wird es die Außenschicht sein, die verstärkt ist; die Innenschicht behält dann ihre Flexibilität, mit der sie Bewegungen des Gelenks ausgleicht. Die Verstärkung kann darin bestehen, dass die betreffende Schicht eine größere Dicke hat, wobei das Material das gleiche sein kann wie das der anderen Schicht. Die betreffende Schicht kann auch faserverstärkt sein, wobei die Fasern vorzugsweise Kurzfasern sind. Versuche haben gezeigt, dass Langfasern schnell brechen.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine Schnittdarstellung einer erfindungsgemäßen Gelenkprothese in Form einer Hüftprothese;
- Fig. 2:: einen vergrößerten Ausschnitt aus Fig. 1;
- Fig. 3 und 4:: die Ansicht aus Fig. 2 bei anderen Ausführungsformen der Erfindung;
- Fig. 5:: eine Draufsicht auf eine erfindungsgemäße Prothese;
- Fig. 6:: die Ansicht aus Fig. 5 bei einer anderen Ausführungsform der Erfindung;
- Fig. 7:: die Ausführungsform aus Fig. 6 in geschnittener Darstellung und mit abgewinkeltem Gleitgelenk; und
- Fig. 8:: die Ansicht aus Fig. 5 bei einer anderen Ausführungsform der Erfindung.

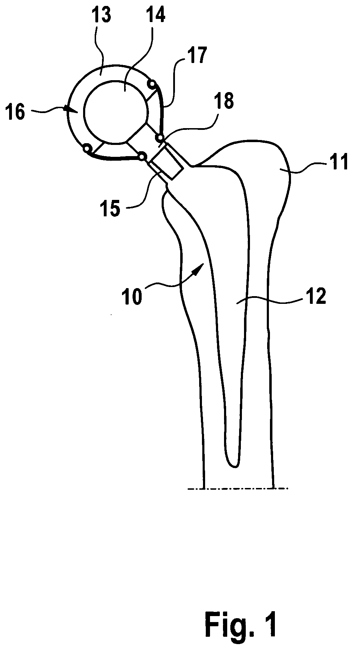

Eine in Figur 1 gezeigte erfindungsgemäße Gelenkprothese in Form einer Hüftprothese 10 umfasst einen mit dem Femur 11 zu verbindenden Schaft 12 und eine in das Acetabulum einzusetzende Pfannenkomponente 13. Über eine KonusSteckverbindung 15 ist ein Kugelkopf 14 mit dem Schaft 12 verbunden. Die Pfannenkomponente 13 bildet die erste Prothesenkomponente und der Kugelkopf 14 zusammen mit dem Hals 18 die zweite Prothesenkomponente im Sinne der Erfindung. Die Außenfläche des Kugelkopfs 14 wirkt zusammen mit der Innenfläche der Pfannenkomponente 13 und bildet ein Gleitgelenk 16.

Wenn das Hüftgelenk bewegt wird, werden durch Reibung zwischen der Außenfläche des Kugelkopfs 14 und der Innenfläche der Pfannenkomponente 13 feine Partikel abgetragen. Um zu verhindern, dass die Partikel in das umliegende Körpergewebe eindringen können, ist die Hüftprothese 10 mit einer künstlichen Gelenkkapsel 17 ausgestattet. Die Gelenkkapsel 17 erstreckt sich vom Hals 18 der Hüftprothese 10 bis zur Außenseite der Pfannenkomponente 13. In Umfangsrichtung sind der Hals 18 und die Pfannenkomponente 13 vollständig von der Gelenkkapsel 17 umgeben. Die Gelenkkapsel 17 schließt mit dem Hals 18 und mit der Pfannenkomponente 13 dicht ab, so dass von dem Gleitgelenk 16 abgetragene Partikel in dem von der Gelenkkapsel 17 umschlossenen Raum gehalten werden und nicht austreten können.

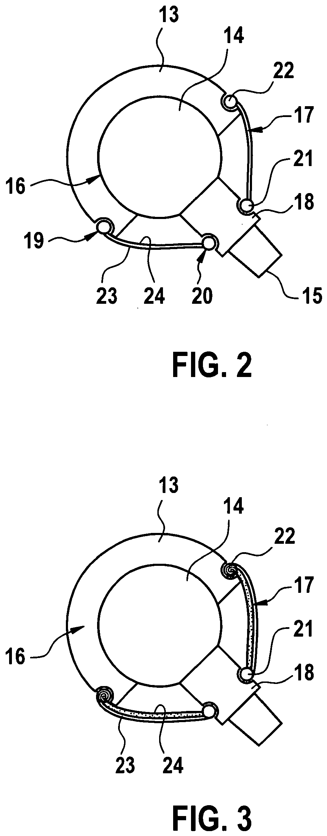

In der Pfannenkomponente 13 und dem Hals 18 sind, wie Fig. 2 zeigt, umlaufende Nuten 19, 20 angeordnet. In die umlaufenden Nuten 19, 20 greifen Wulste 21, 22 ein, die an den beiden Enden der Gelenkkapsel 17 ausgebildet sind. Die Wulste 21, 22 sitzen unter Spannung in den Nuten 19, 20 und schließen dadurch dicht mit den Nuten 19, 20 ab. Die umlaufenden Nuten 19, 20 sind im Querschnitt annähernd halbkreisförmig und damit an die Wulste angepasst. Scharfe Kanten in den Nuten 19, 20 sollten vermieden werden, weil anderenfalls die Gefahr besteht, dass die Gelenkkapsel 17 beschädigt wird. Die Wulste 21, 22 haben einen Durchmesser von ungefähr 3 mm bis 4 mm. Die Wand der Gelenkkapsel 17 ist ungefähr 0,6 mm dick.

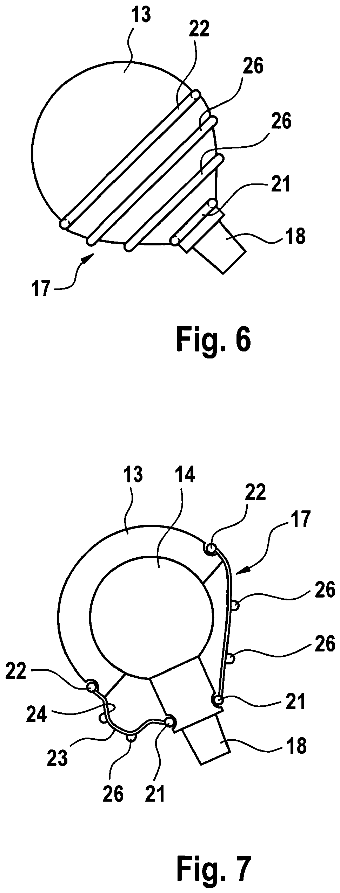

Wenn der Kugelkopf 14 die in Fig. 2 gezeigte Mittelposition in der Pfannenkomponente 13 einnimmt, ist das Material der Gelenkkapsel 17 rundherum leicht gespannt. Knickt der Kugelkopf 14 relativ zu der Pfannenkomponente ein, so erhöht sich die Spannung in der Wand der Gelenkkapsel 17 auf der einen Seite und vermindert sich auf der anderen Seite, siehe Fig. 7. Das Material der Gelenkkapsel 17 ist elastisch dehnbar, so dass die Wand wieder ihren ursprünglichen Zustand einnimmt, wenn das Gelenk in seine Ausgangsposition zurückgekehrt. Beispielsweise kann die Gelenkkapsel 17 aus einem Silikonmaterial bestehen, wie es bei Brustimplantaten Verwendung findet.

Die Wand der Gelenkkapsel 17 besteht aus einer Außenschicht 23 und einer Innenschicht 24. Die Außenschicht 23 und die Innenschicht 24 liegen direkt aufeinander, sind aber außer im Bereich der Wulste 21, 22 nicht miteinander verbunden. Parallel zur Wand der Gelenkkapsel 17 können die Außenschicht 23 und die Innenschicht 24 also relativ zueinander verschoben werden. Um das Verschieben zu erleichtern, kann auf einer oder beiden der aneinander liegenden Flächen eine reibungsmindernde Beschichtung aufgebracht sein. Wenn die Hüftprothese 10 bestimmungsgemäß implantiert ist, steht die Außenschicht 23 in unmittelbarem Kontakt mit umliegendem Körpergewebe. Das Körpergewebe geht im Laufe der Zeit eine Verbindung mit der Außenschicht 23 ein. Um das Anwachsen des Körpergewebes zu erleichtern, ist die Außenschicht 23 mit einer strukturierten Beschichtung versehen. Die Innenschicht 24 kann Bewegungen des Gleitgelenks 16 ausgleichen, ohne dass sie am umliegenden Körpergewebe reibt und dieses reizt.

Die Außenschicht 23 ist, wie unten näher ausgeführt wird, so gestaltet, dass sie sich nach außen wölbt, wenn sich die Spannung in der Wand der Gelenkkapsel 17 nach einem Einknicken des Gelenks vermindert. Da die Außenschicht 23 und die Innenschicht 24 unmittelbar aufeinander liegen, wirken Adhäsionskräfte zwischen beiden, so dass die Innenschicht 24 mit nach außen gezogen wird, wenn sich die Außenschicht 23 nach außen wölbt. Dadurch wird verhindert, dass die Gelenkkapsel 17 zwischen der Pfannenkomponente 13 und dem Kugelkopf 14 eingeklemmt wird und Schaden nimmt.

Bei der in Fig. 3 gezeigten Ausführungsform liegen die Außenschicht 23 und die Innenschicht 24 nicht unmittelbar aufeinander, sondern es ist eine Flüssigkeit in den Zwischenraum zwischen der Außenschicht 23 und der Innenschicht 24 eingebracht. Die Außenschicht 23 ist, wie es durch die Doppellinie angedeutet ist, verstärkt, um sicher auszuschließen, dass die Gelenkkapsel einreißt. Durch die Flüssigkeit wird die Reibung zwischen der Außenschicht 23 und der Innenschicht 24 vermindert und sie können leichter parallel zueinander verschoben werden. Zugleich hat die Flüssigkeit eine Dämpfungsfunktion, durch die beispielsweise Stöße zwischen dem umliegenden Gewebe und der Prothese abgemildert werden. Anstatt mit einer Flüssigkeit kann der Zwischenraum auch mit einem Gel oder einem vergleichbaren Stoff gefüllt sein.

Die Flüssigkeitsschicht in dem Zwischenraum ist so dünn, dass die Außenschicht 23 und die Innenschicht 24 trotz der Flüssigkeit im Zwischenraum durch Kapillarwirkung (vergleichbar mit dem Pleuralspalt) aneinander haften. Wölbt sich die Außenschicht 23 bei einer Biegung des Gelenks nach außen, so wird die Innenschicht 24 mittels kapillarer Kräfte ebenfalls nach außen gezogen.

Die Wulste 21, 22 sind in Fig. 3 dadurch gebildet, dass die Außenschicht 23 und die Innenschicht 24 gemeinsam zu einem Wulst aufgerollt sind. Durch das Aufrollen wird zugleich der Zwischenraum zwischen der Außenschicht 23 und der Innenschicht 24 dicht verschlossen, so dass die Flüssigkeit in dem Zwischenraum gehalten wird und nicht austreten kann.

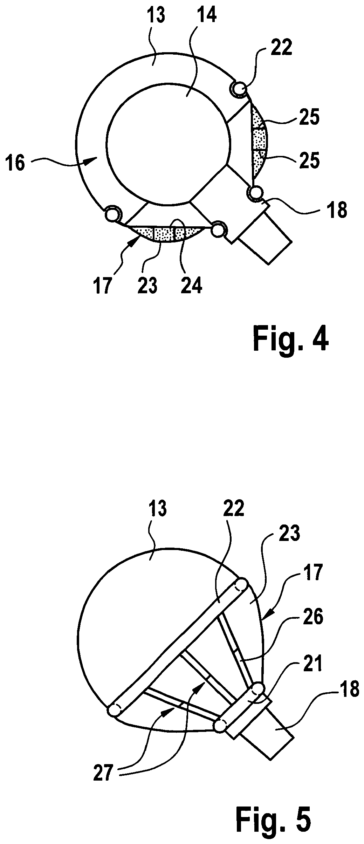

Die Fig. 4 zeigt eine Ausführungsform, bei der die Innenschicht 24 nur zum Teil mit einer Außenschicht versehen ist. Die Außenschicht 23 erstreckt sich über ungefähr 70% der Fläche der Gelenkkapsel 17. Die Außenschicht 23 hat einen größeren Abstand zu der Innenschicht 24 als in den vorangegangenen Beispielen. Der Zwischenraum zwischen der Innenschicht 24 und der Außenschicht 23 ist mit einer Flüssigkeit zur Dämpfung und Verminderung der Reibung gefüllt.

Der Zwischenraum zwischen der Außenschicht 23 und der Innenschicht 24 ist so groß, dass die Schichten nicht mehr durch kapillare Kräfte aneinander haften. Um dennoch eine Kraftübertragung zu ermöglichen, sind Zugelemente 25 zwischen der Außenschicht 23 und der Innenschicht 24 ausgebildet. Wölbt sich die Außenschicht 23 bei einem Einknicken des Gelenks nach außen, so wird über die Zugelemente 25 auch die Innenschicht 24 nach außen gezogen. Die Innenschicht 24 kann nicht zwischen der Gelenkpfanne 13 und dem Kugelkopf 14 eingeklemmt werden.

Fig. 5 zeigt eine Ansicht von außen auf den oberen Abschnitt der Hüftprothese. Die Gelenkkapsel 17 wird über Wulste 21, 22 in Nuten der Gelenkpfanne 13 bzw. des Halses 18 gehalten. Zwischen den Wulsten 21 und 22 erstreckt sich eine Mehrzahl von Verstärkungsstreifen 26. Die Verstärkungsstreifen 26 haben in der Nähe der Wulste 21, 22 eine geringere Dicke und erreichen in der Mitte bei 27 ihre maximale Dicke. Im Querschnitt verjüngen sich die Verstärkungsstreifen 26 von der Basis zum freien Ende und haben eine annähernd dreieckige Form. Knickt nun das Gelenk ein, so vermindert sich auf einer Seite der Abstand zwischen dem Wulst 21 und dem Wulst 22. Aufgrund der Verstärkungsstreifen 26 gleicht die Gelenkkapsel 17 dieser Abstandänderung durch eine Wölbung nach außen aus. Zusammen mit der Außenschicht 23 wird auch die Innenschicht 24 nach außen gezogen, so dass die Gelenkkapsel 17 nicht in dem Gleitgelenk 16 eingeklemmt wird.

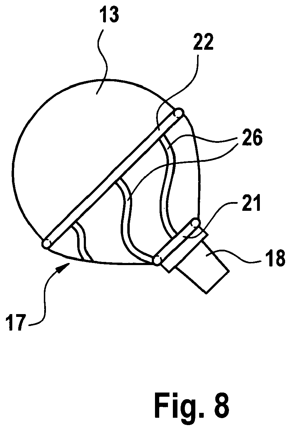

Fig. 8 zeigt eine Ausführungsform, bei der sich die Verstärkungsstreifen nicht geradlinig zwischen den den Wulsten 21, 22 erstrecken, sondern in einer gebogenen S-Form. Damit ist die Gelenkkapsel besonders gut geeignet, um Twistbewegungen zwischen den Prothesenkomponenten 13, 14 auszugleichen.

Bei der Ausführungsform der Fig. 6 erstrecken sich die Verstärkungsstreifen 26 nicht von dem Wulst 21 zu dem Wulst 22, sondern quer dazu in Umfangsrichtung um die Gelenkkapsel 17 herum. Die Wirkung ist die gleiche wie bei den Verstärkungsstreifen der Fig. 5. Die Gelenkkapsel 17 wölbt sich nach außen, wenn das Gelenk einknickt. Das Einknicken des Gelenks ist in der Schnittdarstellung der Fig. 7 veranschaulicht. Auf der rechten Seite vergrößert sich der Abstand zwischen dem Wulst 21 und dem Wulst 22, die Wand der Gelenkkapsel 17 wird unter größere Spannung gesetzt und dehnt sich aus. Auf der linken Seite vermindert sich der Abstand zwischen dem Wulst 21 und dem Wulst 22 und damit auch die Spannung in der Wand der Gelenkkapsel 17. Aufgrund der Verstärkungsstreifen 26 wölbt sich die Außenschicht 23 nach außen und zieht die Innenschicht 24 ebenfalls nach außen.

## Patentansprüche

1. Gelenkprothese mit einer ersten Prothesenkomponente (13) und einer zweiten Prothesenkomponente (14, 18), die über Gelenkflächen zusammenwirken und ein Gleitgelenk (16) bilden, und mit einer Gelenkkapsel (17), die sich von der ersten Prothesenkomponente (13) zu der zweiten Prothesenkomponente (14, 18) erstreckt und das Gleitgelenk (16) umschließt, **dadurch gekennzeichnet, dass** in einem Flächenbereich der Gelenkkapsel (17) eine doppelte Wand vorgesehen ist, in der eine Innenschicht (24) und eine Außenschicht (23) parallel zueinander verschiebbar sind.

2. Gelenkprothese nach Anspruch 1, **dadurch gekennzeichnet, dass** die Innenschicht (24) und die Außenschicht (23) unmittelbar aufeinander liegen.

3. Gelenkprothese nach Anspruch 2, **dadurch gekennzeichnet, dass** auf der Innenschicht (24) und/oder der Außenschicht (23) eine reibungsmindernde Beschichtung aufgebracht ist.

4. Gelenkprothese nach Anspruch 1, **dadurch gekennzeichnet, dass** zwischen der Innenschicht (24) und der Außenschicht (23) eine Flüssigkeitsschicht bzw. Gelschicht vorgesehen ist.

5. Gelenkprothese nach Anspruch 4, **dadurch gekennzeichnet, dass** die Flüssigkeitsschicht bzw. Gelschicht so dünn ist, dass die Innenschicht (24) und die Außenschicht (23) kapillar aneinander haften.

6. Gelenkprothese nach Anspruch 4 oder 5, **dadurch gekennzeichnet, dass** die Innenschicht (24) und die Außenschicht (23) einen Abstand zueinander haben.

7. Gelenkprothese nach Anspruch 6, **dadurch gekennzeichnet, dass** ein oder mehrere Zugelemente (25) zwischen der Innenschicht (24) und der Außenschicht (23) vorgesehen sind.

8. Gelenkprothese nach einem der Ansprüche 1 bis 7, **dadurch gekennzeichnet, dass** am Ende der Gelenkkapsel (17) ein Wulst (21, 22) ausgebildet ist und dass der Wulst (21, 22) in einer Vertiefung (19, 20) einer der beiden Prothesenkomponente gehalten ist.

9. Gelenkprothese nach Anspruch 8, **dadurch gekennzeichnet, dass** der Wulst (21, 22) durch Aufrollen der Gelenkkapsel (17) gebildet ist.

10. Gelenkprothese nach einem der Ansprüche 1 bis 9, **dadurch gekennzeichnet, dass** die Außenschicht (23) einen oder mehrere Verstärkungsstreifen (26) aufweist.

11. Gelenkprothese nach Anspruch 10, **dadurch gekennzeichnet, dass** ein Verstärkungsstreifen (26) als Verstärkungsring ausgebildet ist, der das Gleitgelenk (16) in Umfangsrichtung umschließt.

12. Gelenkprothese nach Anspruch 10 oder 11, **dadurch gekennzeichnet, dass** ein Verstärkungsstreifen (26) sich zwischen zwei gegenüberliegenden Enden der Gelenkkapsel (17) erstreckt.

13. Gelenkprothese nach einem der Ansprüche 10 bis 12, **dadurch gekennzeichnet, dass** die Verstärkungsstreifen (26) im Querschnitt annähernd dreieckig sind.

14. Gelenkprothese nach einem der Ansprüche 1 bis 13, **dadurch gekennzeichnet, dass** die Wand der Gelenkkapsel aus einem elastisch dehnbaren Material besteht.

15. Gelenkprothese nach einem der Ansprüche 1 bis 14, **dadurch gekennzeichnet, dass** die Außenschicht (23) auf ihrer Außenseite eine Struktur aufweist, die das Anwachsen von Körpergewebe erleichtert.

## Claims

1. Joint prosthesis with a first prosthesis component (13) and a second prosthesis component (14, 18) which interact via joint surfaces and form a sliding joint (16), and with a joint capsule (17) which extends from the first prosthesis component (13) to the second prosthesis component (14, 18) and surrounds the sliding joint (16), **characterized in that** in a surface region of the joint capsule (17) a double wall is provided in which an inner layer (24) and an outer layer (23) are displaceable parallel to each other.

2. Joint prosthesis according to Claim 1, **characterized in that** the inner layer (24) and the outer layer (23) lie directly on each other.

3. Joint prosthesis according to Claim 2, **characterized in that** a friction-reducing coating is applied on the inner layer (24) and/or the outer layer (23).

4. Joint prosthesis according to Claim 1, **characterized in that** a liquid layer or gel layer is provided between the inner layer (24) and the outer layer (23).

5. Joint prosthesis according to Claim 4, **characterized in that** the liquid layer or gel layer is so thin that the inner layer (24) and the outer layer (23) adhere to each other with a capillary action.

6. Joint prosthesis according to Claim 4 or 5, **characterized in that** the inner layer (24) and the outer layer (23) are at a distance from each other.

7. Joint prosthesis according to Claim 6, **characterized in that** one or more tension elements (25) are provided between the inner layer (24) and the outer layer (23).

8. Joint prosthesis according to one of Claims 1 to 7, **characterized in that** a bead (21, 22) is formed at the end of the joint capsule (17), and **in that** the bead (21, 22) is held in a depression (19, 20) of one of the two prosthesis components.

9. Joint prosthesis according to Claim 8, **characterized in that** the bead (21, 22) is formed by rolling up the joint capsule (17).

10. Joint prosthesis according to one of Claims 1 to 9, **characterized in that** the outer layer (23) has one or more reinforcing strips (26).

11. Joint prosthesis according to Claim 10, **characterized in that** one reinforcing strip (26) is designed as a reinforcing ring which surrounds the sliding joint (16) in the circumferential direction.

12. Joint prosthesis according to Claim 10 or 11, **characterized in that** a reinforcing strip (26) extends between two opposite ends of the joint capsule (17).

13. Joint prosthesis according to one of Claims 10 to 12, **characterized in that** the reinforcing strips (26) are approximately triangular in cross section.

14. Joint prosthesis according to one of Claims 1 to 13, **characterized in that** the wall of the joint capsule is composed of an elastically stretchable material.

15. Joint prosthesis according to one of Claims 1 to 14, **characterized in that** the outer side of the outer layer (23) has a structure which facilitates the growing of body tissue thereon.

## Revendications

1. Prothèse d'articulation avec un premier composant de prothèse (13) et un deuxième composant de prothèse (14, 18), qui coopèrent par des surfaces d'articulation et forment une articulation glissante (16), et avec une capsule articulaire (17), qui s'étend du premier composant de prothèse (13) au deuxième composant de prothèse (14, 18) et entoure l'articulation glissante (16), **caractérisée en ce qu'**il est prévu dans une zone de surface de la capsule articulaire (17) une double paroi, dans laquelle une couche intérieure (24) et une couche extérieure (23) sont déplaçables parallèlement l'une à l'autre.

2. Prothèse d'articulation selon la revendication 1, **caractérisée en ce que** la couche intérieure (24) et la couche extérieure (23) reposent directement l'une sur l'autre.

3. Prothèse d'articulation selon la revendication 2, **caractérisée en ce qu'**un revêtement réduisant le frottement est déposé sur la couche intérieure (24) et/ou sur la couche extérieure (23).

4. Prothèse d'articulation selon la revendication 1, **caractérisée en ce qu'**il est prévu une couche de liquide ou une couche de gel entre la couche intérieure (24) et la couche extérieure (23).

5. Prothèse d'articulation selon la revendication 4, **caractérisée en ce que** la couche de liquide ou la couche de gel est tellement mince que la couche intérieure (24) et la couche extérieure (23) adhèrent l'une à l'autre par capillarité.

6. Prothèse d'articulation selon la revendication 4 ou 5, **caractérisée en ce que** la couche intérieure (24) et la couche extérieure (23) présentent une distance l'une de l'autre.

7. Prothèse d'articulation selon la revendication 6, **caractérisée en ce qu'**il est prévu un ou plusieurs élément(s) de traction (25) entre la couche intérieure (24) et la couche extérieure (23).

8. Prothèse d'articulation selon l'une quelconque des revendications 1 à 7, **caractérisée en ce qu'**un bourrelet (21, 22) est formé à l'extrémité de la capsule articulaire (17) et **en ce que** le bourrelet (21, 22) est maintenu dans un creux (19, 20) d'un des deux composants de prothèse.

9. Prothèse d'articulation selon la revendication 8, **caractérisée en ce que** le bourrelet (21, 22) est formé par enroulement de la capsule articulaire (17).

10. Prothèse d'articulation selon l'une quelconque des revendications 1 à 9, **caractérisée en ce que** la couche extérieure (23) présente une ou plusieurs bande(s) de renforcement (26).

11. Prothèse d'articulation selon la revendication 10, **caractérisée en ce qu'**une bande de renforcement (26) est réalisée sous la forme d'un anneau de renforcement, qui entoure l'articulation glissante (16) en direction périphérique.

12. Prothèse d'articulation selon la revendication 10 ou 11, **caractérisée en ce qu'**une bande de renforcement (26) s'étend entre deux extrémités opposées de la capsule articulaire (17).

13. Prothèse d'articulation selon l'une quelconque des revendications 10 à 12, **caractérisée en ce que** les bandes de renforcement (26) sont approximativement triangulaires en section transversale.

14. Prothèse d'articulation selon l'une quelconque des revendications 1 à 13, **caractérisée en ce que** la paroi de la capsule articulaire se compose d'un matériau élastiquement extensible.

15. Prothèse d'articulation selon l'une quelconque des revendications 1 à 14, **caractérisée en ce que** la couche extérieure (23) présente sur son côté extérieur une structure, qui facilite la croissance de tissu corporel.
